# EUROPÄISCHE PATENTANMELDUNG

(11) **EP 2 823 835 A2**
(43) Veröffentlichungstag der Anmeldung: **14.01.2015**
(21) Anmeldenummer: 14162316.5
(22) Anmeldetag: 28.03.2014
(51) Int. Cl.: A61M 5/165, A61M 5/168

(54) **Druckmesszelle zur Verwendung in einem Infusions- oder Injektionssystem**

(30) Priorität: 24.06.2013 DE 102013106582
(71) Anmelder: Ulrich GmbH & Co. KG, 89081 Ulm (DE)
(72) Erfinder: Fox, Frederik, 89081 Ulm (DE); Striggow, Uwe, 89081 Ulm (DE); Kolich, Florian, 89081 Ulm (DE); Philipp, Daniel, 89081 Ulm (DE)
(74) Vertreter: Charrier, Rapp & Liebau

(57) **Zusammenfassung**

Die Erfindung betrifft eine Druckmesszelle zur Verwendung in einem Infusions- oder Injektionssystem zur Injektion eines Fluids, mit einem Gehäuse (1), in dem wenigstens eine Filtereinheit (4), durch welche das unter Druck stehende Fluid geleitet wird, sowie wenigstens ein Druckaufnehmer (3) zur Erfassung des Drucks des aus der Filtereinheit (4) strömenden Fluids angeordnet sind. Eine solche Druckmesszelle ist druckstabil, verursacht nur einen geringen Druckabfall im Schlauchsystem des Infusions- oder Injektionssystem, ist kostengünstig herstellbar und ermöglicht eine einfache Montage und Handhabung beim Einsetzen des Schlauchssystems in eine Schlauchpumpe.

## Beschreibung

Die Erfindung betrifft eine Druckmessezelle zur Verwendung in einem Infusions- oder Injektionssystem zur Injektion eines Fluids, wobei die Druckmesszelle ein Gehäuse umfasst, , in dem wenigstens eine Filtereinheit (4), durch welche das unter Druck stehende Fluid geleitet wird, sowie ein Druckaufnehmer (3) zur Erfassung des Drucks des aus der Filtereinheit (4) strömenden Fluids angeordnet sind.

In Infusions- oder Injektionssystem zur Injektion einer Flüssigkeit werden zur Erfassung des Drucks der zu injizierenden Flüssigkeit Druckmesszellen verwendet, welche bspw. in einem Injektionsschlauch angeordnet sind, in dem die Flüssigkeit zu einer Injektionskanüle gefördert wird. So zeigt beispielsweise die Offenlegungsschrift DE 19900937 A1 einen Injektor zur Applizierung von Flüssigkeiten, insbesondere von Kontrastmitteln für die Röntgen- oder Kernspintomographie, mit einem Schlauch sowie einer in Umfangsrichtung zumindest teilweise von dem Schlauch umschlungenen Rollenpumpe zur Förderung der Flüssigkeit von einem Vorratsgefäß zu einer Kanüle, wobei eine durch eine in der Schlauchwandung ausgebildete Öffnung mit dem Inneren des Schlauches (3) verbundene Druckkammer vorhanden ist, die ein unter der Wirkung des Flüssigkeitsdruckes verstellbares Bauteil aufweist, das auf einen Drucksensor einwirkt.

Aus der europäischen Patentschrift EP 2011541 B1 ist ein Schlauchsystem für einen Injektor zum intravenösen Injizieren von Kontrastmitteln und Kochsalzlösung bekannt, mit einem mit einer Pumpe gekoppelten Pumpenschlauch zum Fördern des Kontrastmittels bzw. der Kochsalzlösung, in dem ein Drucksensor sowie getrennt davon ein Partikelfilter hintereinander angeordnet sind.

Nachteilig bei einem solchen System, bei dem die Druckmesszelle und der Partikelfilter voneinander getrennt sind, ist der Kostenfaktor. So haben der Drucksensor und der Partikelfilter eigene Gehäuse, welche die Herstellungskosten des Systems erhöhen. Zudem ist die Montage der Einzelteile aufwendiger, da mehr Arbeitsschritte beim Zusammenbau des Schlauchsystems getätigt werden müssen. Ein weiterer Nachteil ist die geringe Druckstabilität des verwendeten Partikelfilters, der üblicherweise als Tellerfilter ausgebildet ist. Ungünstig ist weiterhin der erhöhte Druckabfall zwischen dem Filtereingang und dem Filterausgang des Partikelfilters, der sich durch die relativ kleine Filterfläche ergibt, die dem Querschnitt des Pumpenschlauchs entspricht. Nachteilig ist ferner die Handhabung beim Einlegen des Pumpenschlauchs in die Pumpe.

Der Erfindung liegt daher die Aufgabe zugrunde, eine Druckmesszelle zur Verwendung in einem Infusions- oder Injektionssystem bereit zu stellen, welche kostengünstiger herstellbar ist, eine einfache Montage ermöglicht, möglichst druckstabil ist und nur einen geringen Druckabfall im Schlauchsystem des Infusions- oder Injektionssystem verursacht sowie eine möglichst einfache Handhabung beim Einsetzen des Schlauchssystems in eine Schlauchpumpe gewährleistet.

Diese Aufgabe wird bei einer Druckmesszelle mit einem Gehäuse, in dem ein Druckaufnehmer zur Erfassung des Drucks des durchströmenden Fluids angeordnet ist, erfindungsgemäß dadurch gelöst, dass in dem Gehäuse eine Filtereinheit angeordnet ist, durch welche das Fluid strömt. Dadurch sind der Druckaufnehmer und der Partikelfilter in einem Bauteil mit einem gemeinsamen Gehäuse integriert. Dies ermöglicht eine kostengünstigere Herstellung, weil im Vergleich zu den Systemen des Stand der Technik, in denen der Druckaufnehmer und der Partikelfilter als getrennte Bauteile ausgebildet und hintereinander im Injektionsschlauch angeordnet werden, ein Gehäuseteil wegfällt. Auch die Montage des Injektionssystems erleichtert sich durch den Einsatz der erfindungsgemäßen Druckmesszelle, da nur die Druckmesszelle mit der integrierten Filtereinheit in einem Arbeitsschritt in den Injektionsschlauch eingesetzt werden muss, wohingegen bei den bekannten Systemen nacheinander der Drucksensor und der Partikelfilter eingesetzt werden müssen. Weiterhin wird eine einfache Handhabung beim Einsetzen des Schlauchssystems in eine Schlauchpumpe gewährleistet, da in dem Schlauchsystem nun nur noch ein Gehäuseteil enthalten ist, in dem sowohl die Filtereinheit als auch der Druckaufnehmer untergebracht sind.

Die Filtereinheit ist bevorzugt als längliche Filterkerze mit einem Kerzengehäuse und einem Filtersieb ausgebildet, welche in eine Gehäusebohrung einsteckbar ist, wobei das Kerzengehäuse ein Filtersieb trägt und eine Kerzenöffnung aufweist, durch welche das unter Druck stehende Fluid in die Filterkerze einströmt. Dadurch kann der Strömungsquerschnitt durch die Filtereinheit im Vergleich zu den bekannten Partikelfiltern, bei denen der Strömungsquerschnitt dem Querschnitt des Injektionsschlauchs entspricht, erhöht werden, weil die effektive Filterfläche der länglichen Filterkerze größer als der Schlauchquerschnitt ausgebildet werden kann. Der in der Filtereinheit erzeugte Druckabfall wird auf diese Weise minimiert und die Druckstabilität wird verbessert.

In einem bevorzugten Ausführungsbeispiel der erfindungsgemäßen Druckmesszelle ist die Filtereinheit in einer Gehäusebohrung in dem Gehäuse angeordnet, wobei zwischen der Innenfläche der Gehäusebohrung und der Außenseite der in das Gehäuse eingesteckten Filterkerze eine mit einer Auslassöffnung des Gehäuses in Verbindung stehende Kammer ausgebildet ist, in welche das Fluid aus der Filtereinheit herausströmt. Im Kerzengehäuse sind zweckmäßig vom Filtersieb abgedeckte Ausnehmungen ausgebildet, durch welche das Fluid aus der Filtereinheit herausströmt. Das Filtersieb ist zweckmäßig aus einem Filtergewebe mit einer Maschenweite von 10 - 15 µm gebildet und ist, je nach Einsatzzweck in Abhängigkeit des zu injizierenden Fluids, hydrophil oder hydrophob.

Bevorzugt ist die Filterkerze so geformt, dass die Stromdichte des aus der Filtereinheit in die Kammer ausströmenden Fluids längs der Filterkerze über ihre gesamte Länge im Wesentlichen konstant ist. Hierfür sind zweckmäßig im Kerzengehäuse in Längsrichtung der Filterkerze verlaufende Strömungskanäle ausgebildet, in denen das Fluid geführt wird, wobei die Strömungskanäle einerseits von der Außenfläche des Kerzengehäuses und andererseits vom Filtersieb begrenzt sind. Eine über die gesamte Länge der Filterkerze in Strömungsrichtung konstante Stromdichte des ausströmenden Fluids kann dabei erzielt werden, indem sich der Strömungsquerschnitt der Strömungskanäle in Strömungsrichtung kontinuierlich verringert.

Der Druckaufnehmerist zweckmäßig mit einem Drucksensor gekoppelt, bspw. einem piezoresistiven oder piezoelektrischen Drucksensor.

Die Filtereinheit ist bevorzugt mittels einem in eine stirnseitige Einlassöffnung des Gehäuses einschiebbaren Stopfen oder eine Buchse in dem Gehäuse gesichert. Der Stopfen bzw. die Buchse weist ein rohrförmiges Anschlussstück für eine Schlauchleitung des Infusions- oder Injektionssystem sowie einen Durchgangskanal auf, durch den das Fluid in die Filtereinheit geleitet wird. In einem zweckmäßigen Ausführungsbeispiel ist die Filtereinheit austauschbar in der Gehäusebohrung angeordnet.

Zweckmäßig verfügen die Filtereinheit und die Innenflächen der Gehäusebohrung über miteinander korrespondierende Führungsmittel wie z.B. Führungsnuten und Führungsrippen, über welche die Filterkerze beim Einsetzen in das Gehäuse geführt in die Gehäusebohrung eingeschoben oder zum Austausch gegen eine neue Filtereinheit aus dem Gehäuse heraus gezogen werden kann. Dadurch wird beim Herausziehen bzw. Hineinschieben der Filtereinheit ein Verkanten oder Verklemmen verhindert.

Diese und weitere Vorteile der Erfindung ergeben sich aus dem nachfolgend unter Bezugnahme auf die begleitenden Zeichnungen näher beschriebenen Ausführungsbeispiel. Die Zeichnungen zeigen:
- **Figur 1**: Perspektivische Darstellung einer erfindungsgemäßen Druckmesszelle;
- **Figur 2**: Draufsicht auf die Druckmesszelle von Figur 1;
- **Figur 3**: Seitenansicht auf die hintere Stirnseite der Druckmesszelle von Figur 2;
- **Figur 4**: Querschnitt durch die Druckmesszelle von Figur 3 längs der Ebene O-O;
- **Figur 5**: Querschnitt durch die Druckmesszelle von Figur 3 längs der Ebene P-P;
- **Figur 6**: Perspektivische Darstellung der Filtereinheit der Druckmesszelle von Figur 1;
- **Figur 7**: Seitenansicht auf die vordere Stirnfläche der Filtereinheit von Figur 6;
- **Figur 8**: Querschnitt durch die Filtereinheit von Figur 7 längs der Ebene L-L;
- **Figur 9**: Querschnitt durch die Filtereinheit von Figur 7 längs der Ebene K-K;
- **Figur 10**: Perspektivische Darstellung einer zweiten Ausführungsform einer Filtereinheit für eine erfindungsgemäße Druckmesszelle;
- **Figur 11**: Seitenansicht auf die vordere Stirnfläche der Filtereinheit von Figur 10;
- **Figur 12**: Schnittdarstellung der Filtereinheit von Figur 11 längs der Ebene AG-AG;
- **Figur 13**: Querschnittsdarstellung der Filtereinheit von Figur 11 längs der Ebene AF-AF;
- **Figur 14**: Perspektivische Darstellung eines Einsatzes einer dritten Ausführungsform einer Filtereinheit für eine erfindungsgemäße Druckmesszelle;
- **Figur 15**: Seitenansicht auf die Stirnfläche des Einsatzes von Figur 14;
- **Figur 16**: Schnittdarstellung des Einsatzes von Figur 15 längs der Ebene AC-AC;
- **Figur 17**: Schnittdarstellung des Einsatzes von Figur 15 längs der Ebene AE-AE;
- **Figur 18**: Schnittdarstellung des Einsatzes von Figur 15 längs der Ebene AJ-AJ;
- **Figur 19**: Draufsicht auf den Einsatz von Figur 14;

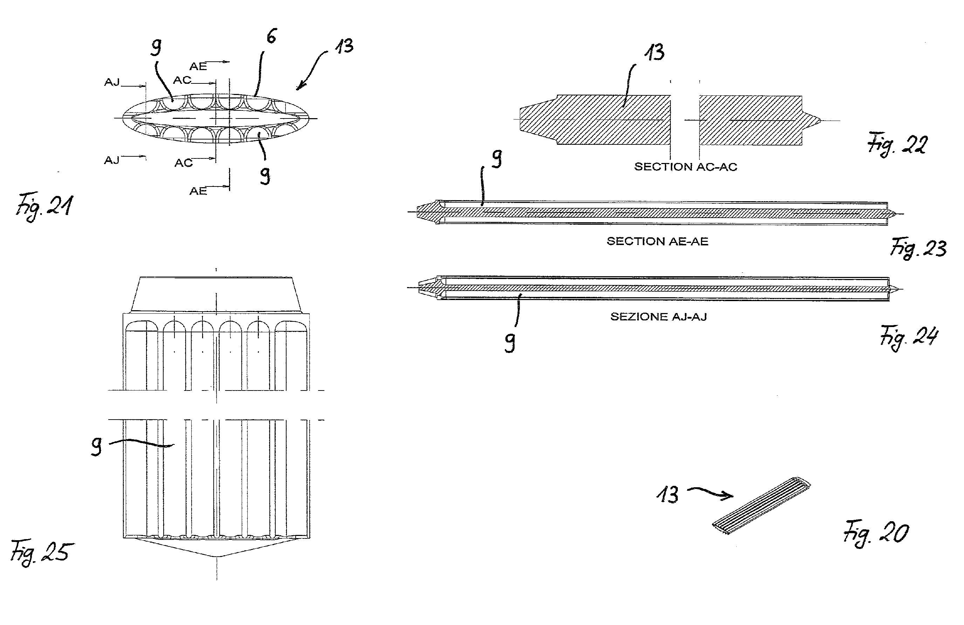

- **Figur 20**: Perspektivische Darstellung eines Einsatzes für eine vierte Ausführungsform einer Filtereinheit für eine erfindungsgemäße Druckmesszelle;
- **Figur 21**: Seitenansicht auf die Stirnfläche des Einsatzes von Figur 20;
- **Figur 22**: Schnittdarstellung des Einsatzes von Figur 21 längs der Ebene AC-AC;
- **Figur 23**: Schnittdarstellung des Einsatzes von Figur 21 längs der Ebene AE-AE;
- **Figur 24**: Schnittdarstellung des Einsatzes von Figur 21 längs der Ebene AJ-AJ;
- **Figur 25**: Draufsicht auf den Einsatz Filtereinheit von Figur 20;
- **Figur 26**: Darstellung eines weiteren Ausführungsbeispiels einer erfindungsgemäßen Druckmesszelle in einer Querschnittsdarstellung (Figur 26a), einer Draufsicht (Figur 26b) sowie Schnittdarstellungen längs der Ebenen Q-Q (Figur 26c), R-R (Figur 26d) und S-S (Figur 26e).

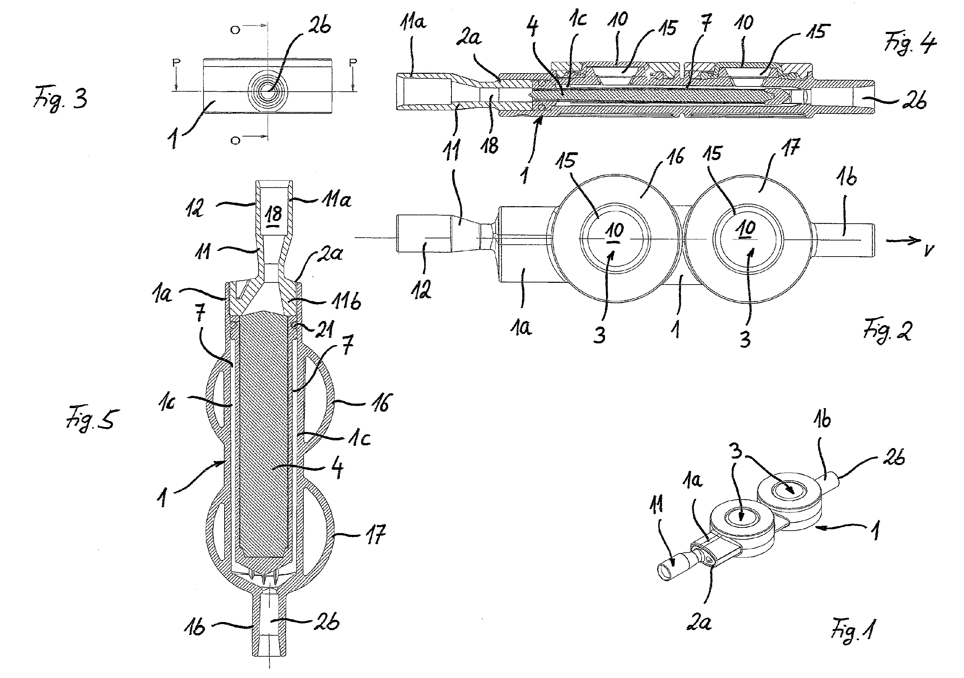

In den Figuren 1 bis 5 ist eine erfindungsgemäße Druckmesszelle in einer perspektivischen Ansicht gezeigt. Diese Druckmesszelle dient zur Verwendung in einem Infusions- oder Injektionssystem zur Injektion eines Fluids, beispielsweise zur intravenösen Injektion eines Kontrastmittels. Die Druckmesszelle weist ein (einziges) Gehäuse 1 auf, welches zweckmäßig als Kunststoffspritzgussteil ausgebildet ist. Das Gehäuse weist eine Gehäusebohrung 1c mit einer Einlassöffnung 2a an seinem vorderen stirnseitigen Ende 1a und einer Auslassöffnung 2b an seinem hinteren stirnseitigen Ende 1b auf. Das hintere Ende 1b des Gehäuses 1 ist rohrförmig ausgebildet und dient zum Anschluss einer Schlauchleitung eines Injektionsschlauchs des Infusions- oder Injektionssystems. Das vordere Ende 1a des Gehäuses 1 ist ebenfalls rohrförmig mit einem ovalen Rohrquerschnitt ausgebildet.

In dem Gehäuse 1 ist wenigstens ein Druckaufnehmer 3 sowie wenigstens eine Filtereinheit 4 angeordnet. Bei dem Druckaufnehmer 3 handelt es sich bevorzugt um eine einer Membran 10, bspw. eine Silikon-Membran. In dem hier gezeigten Ausführungsbeispiel sind zwei solcher Druckaufnehmer 3 in Form von Membranen 10 vorgesehen, welche in Membranöffnungen 15 der Wandung des Gehäuses 1 angeordnet sind. Diese Membranöffnungen 15 sind dabei in zwei tellerförmigen Gehäuseteilen 16 und 17 des Gehäuses 1 an dessen Oberseite angeordnet und stehen mit der Gehäusebohrung 1c in Verbindung (Figur 4).

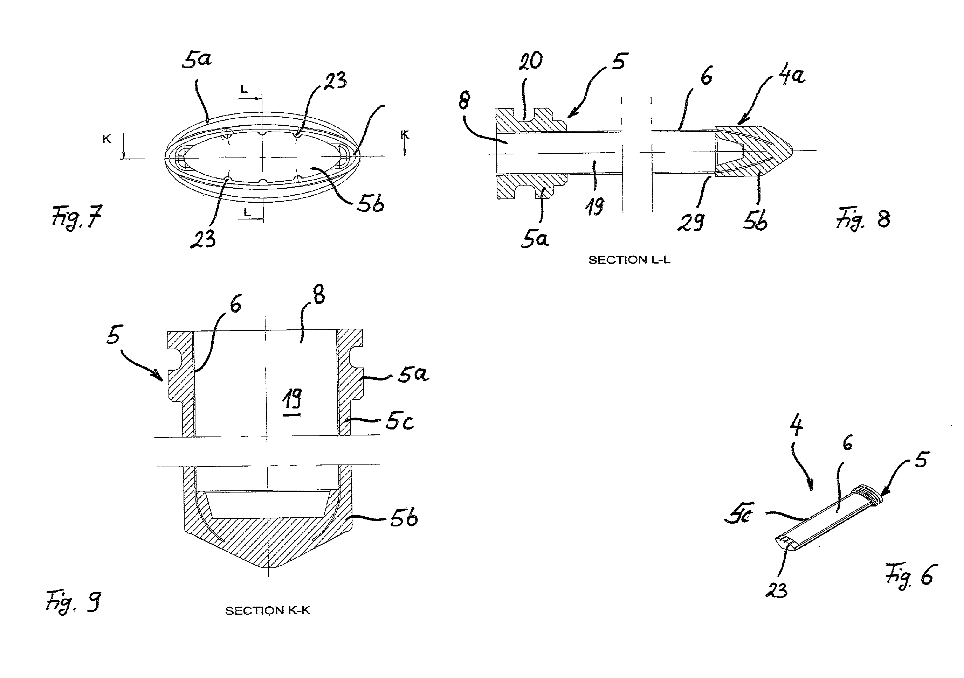

Die in der Gehäusebohrung 1c angeordnete Filtereinheit 4 ist in den Schnittdarstellungen der Figuren 4 und 5 zu erkennen und ist in den Figuren 6 - 9 im Detail dargestellt. Die Filtereinheit 4 ist in der Gehäusebohrung 1c angeordnet und dort mittels eines herausziehbaren Stopfens 11 fixiert und gegen Herausrutschen gesichert. Der Stopfen 11 weist eine Durchgangsbohrung 18 sowie ein vorderes Ende 11a und ein hinteres Ende 11b auf (Figur 5). Das vordere Ende 11a des Stopfens 11 ist als rohrförmiges Anschlussstück 12 zur Verbindung mit einer Schlauchleitung des Infusions- oder Injektionssystems ausgebildet. Das hintere Ende 11b des Stopfens 11 ist komplementär zur ovalen Form des vorderen Endes 1a des Gehäuses 1 geformt und ragt in die Eingangsöffnung 2a des Gehäuses 1. Das hintere Ende 11b des Stopfens 11 ist dort zur Sicherung der Filtereinheit 4 zweckmäßig eingeklemmt oder mittels Rastmittel verrastet.

Die Filtereinheit 4 kann entweder in der Gehäusebohrung 1c befestigt sein, bspw. durch Verkleben, oder sie ist austauschbar darin angeordnet. Eine austauschbare Anordnung ermöglicht ein Wechseln einer verbrauchten Filtereinheit, falls diese verstopft sein sollte. Zum Wechseln einer verbrauchten Filtereinheit 4 kann der Stopfen 11 aus der Gehäuseöffnung 2a herausgezogen werden, um den Zugang zur Filtereinheit 4 freizugeben, welche dann aus der Gehäusebohrung 1c herausgezogen und durch eine neue Filtereinheit ersetzt werden kann.

Die in den Figuren 6 - 9 dargestellte Filtereinheit 4 ist als längliche Filterkerze 4a mit einem hohlen Kerzengehäuse 5 und einem Filtersieb 6 ausgestaltet. Das Kerzengehäuse 5 weist einen vorderen Abschnitt 5a, einen hinteren Abschnitt 5b und einen den vorderen Abschnitt 5a und den hinteren Abschnitt 5b verbindenden Längsabschnitt 5c auf. Der Längsabschnitt 5c wird dabei durch wenigstens zwei sich in Längsrichtung der Filterkerze 4 erstreckende Streben 5c gebildet, welche zweckmäßig einstückig als Kunststoffspritzgussteil mit dem vorderen Abschnitt 5a und dem hinteren Abschnitt 5b des Kerzengehäuses 5 verbunden sind. Im mittleren Abschnitt weist das Kerzengehäuse 5 Ausnehmungen auf, welche von dem Filtersieb 6 überdeckt sind.

Bei dem Filtersieb 6 handelt es sich bevorzugt um ein Filtergewebe mit einer Maschenweite von 10 - 15 µm. Das Filtersieb 6 ist zweckmäßig im vorderen Abschnitt 5a, im hinteren Abschnitt 5b und im mittleren Abschnitt 5c mit dem Kerzengehäuse 5 vergossen und wird dadurch von dem Kerzengehäuse 5 getragen. Im vorderen Abschnitt 5a ist eine Kerzenöffnung 8 vorgesehen, welche mit dem Innenraum 19 des hohlen Kerzengehäuses 5 in Verbindung steht. Wenn die Filtereinheit 4 in das Innere des Gehäuses 1 der Druckmesszelle eingesetzt ist, wie beispielsweise in Figur 5 dargestellt, steht die Kerzenöffnung 8 in Verbindung mit der Bohrung 18 des Stopfens 11. Dadurch kann ein zu injizierendes Fluid, welches über einen Injektionsschlauch dem daran angeschlossenen Stopfen 11 zugeführt wird, durch die Bohrung 18 in dem Stopfen 11 und durch die Kerzenöffnung 8 in den Innenraum 19 der Filterkerze 4a einströmen und von dort durch das Filtersieb 6 herausströmen.

Die Filtereinheit 4 mit der länglichen Filterkerze 4a wird in das Innere des Gehäuses 1 mit dem hinteren Abschnitt 5b des Kerzengehäuses 5 voraus eingeschoben und dort mittels des Stopfens 11 fixiert, wie in Figur 5 dargestellt. Der vordere Abschnitt 5a des Kerzengehäuses 5 weist an seinem Außenumfang einen überstehenden Flansch mit einer Ringnut 20 auf. In diese Ringnut 20 wird zur Abdichtung ein O-Ring 21 eingelegt. Der Außendurchmesser des flanschförmigen Abschnitts 5a des Kerzengehäuses 5 entspricht dabei dem Innendurchmesser der Gehäusebohrung 1c, in die die Filtereinheit 4 eingesetzt wird. Der Durchmesser des mittleren Abschnitts 5c und des hinteren Abschnitts 5b der Filterkerze 4a ist kleiner als der Außendurchmesser des vorderen Abschnitts 5a bzw. des Innendurchmessers der Gehäusebohrung 1c, so dass sich zwischen der Außenfläche der Filterkerze 4a im mittleren Abschnitt 5c und im hinteren Abschnitt 5b sowie der Innenfläche der Gehäusebohrung 1c eine Kammer 7 ausbildet. Das aus dem Innenraum 19 der Filterkerze 4a ausströmende Fluid strömt in diese Kammer 7. Die Kammer 7 steht über die Membranöffnungen 15 mit den Membranen 10 der Druckaufnehmer 3 in Kontakt. Der Druck des Fluids, welches durch die Filtereinheit in die Kammer 7 strömt, wird dadurch auf die Membrane 10 der Druckaufnehmer 3 übertragen. Die Druckaufnehmer 3 sind jeweils an einen nicht dargestellten Drucksensor gekoppelt, der die vom Fluiddruck abhängige Verformung der Membrane 10 erfasst und daraus den Fluiddruck ermittelt. Der Drucksensor kann dadurch den Druck des Fluids erfassen und mittels eines nicht dargestellten Anzeigegeräts zur Anzeige bringen.

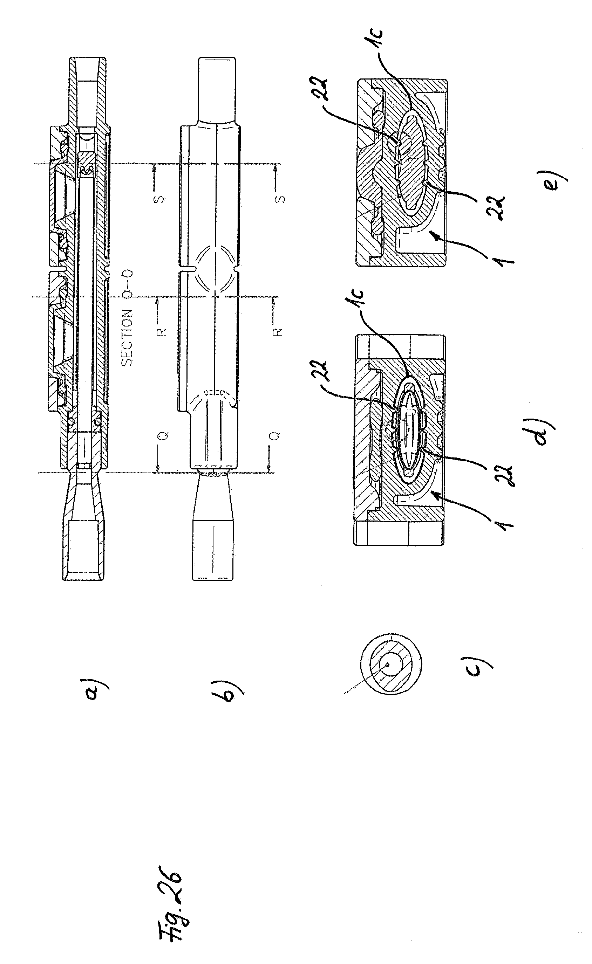

In Figur 26 ist ein weiteres Ausführungsbeispiel einer erfindungsgemäßen Druckmesszelle dargestellt, welches im Wesentlichen dem Ausführungsbeispiel der Figuren 1 - 5 entspricht. Aus den Schnittdarstellungen der Figuren 26d und 26e geht hervor, dass die Innenfläche der Gehäusebohrung 1c an ihrer Oberseite und an ihrer Unterseite zackenförmig vorstehende Stege 22 aufweist. Diese zackenförmigen Stege 22 ragen in den Innenraum der Gehäusebohrung 1c und erstrecken sich im Wesentlichen über die gesamte Länge des Gehäuses 1. Am hinteren Abschnitt 5b des Kerzengehäuses 5 sind in entsprechender Anzahl und an entsprechender Stelle mit diesen Stegen 22 korrespondierende Nuten 23 vorgesehen. Diese Nuten 23 sind in der Darstellung von Figur 6 und Figur 7 zu erkennen. Beim Einschieben der Filtereinheit 4 in die Gehäusebohrung 1c greifen die zackenförmigen Stege 22 in die korrespondierenden Nuten 23 am Kerzengehäuse 5 ein. Dadurch wird beim Einschieben der Filtereinheit 4 in die Gehäusebohrung 1c eine sichere Führung und ein passgenauer Sitz der Filterkerze 4a im Gehäuse 1 gewährleistet.

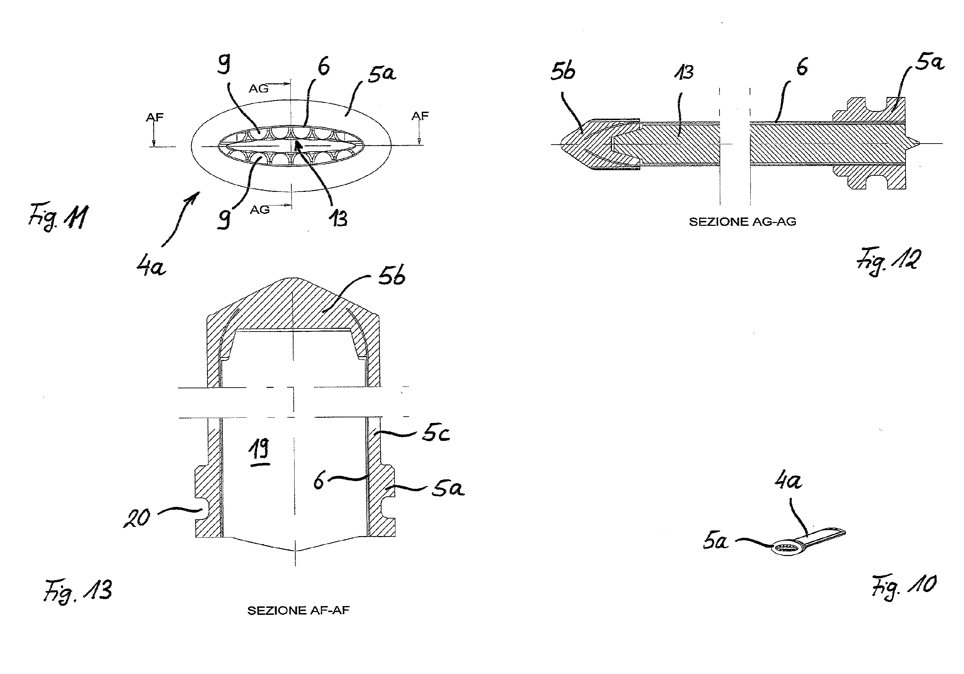

Die Figuren 10 - 13 zeigen eine zweite Ausführungsform einer Filtereinheit 4 zur Verwendung in der erfindungsgemäßen Druckmesszelle. Diese entspricht im Wesentlichen der Filtereinheit des Ausführungsbeispiels der Figuren 6 bis 9, weshalb sich entsprechende Teile mit denselben Bezugszeichen bezeichnet sind. Ergänzend zu dem Ausführungsbeispiel der Figuren 6 bis 9 weist die Filtereinheit 4 des Ausführungsbeispiels der Figuren 10 bis 13 einen Einsatz 13 auf, der in den Innenraum 19 des Kerzengehäuses 5 eingeschoben ist. Der Einsatz 13 weist, wie aus der Darstellung von Figur 11 ersichtlich, an seinem Außenumfang nebeneinander angeordnete und sich in Längsrichtung des Einsatzes 13 erstreckende Strömungskanäle 9 auf. Die Strömungskanäle 9 werden dabei einerseits von der mit halbrohrförmigen Einschnitten versehenen Außenfläche des Einsatzes 13 und andererseits von dem darüber gelegten Filtersieb 6 begrenzt. Das in die Filterkerze 4a einströmende Fluid strömt durch die Strömungskanäle 9 in Längsrichtung der Filterkerze 4a und von dort durch das Filtersieb 6 aus der Filterkerze 4a heraus und in die Kammer 7.

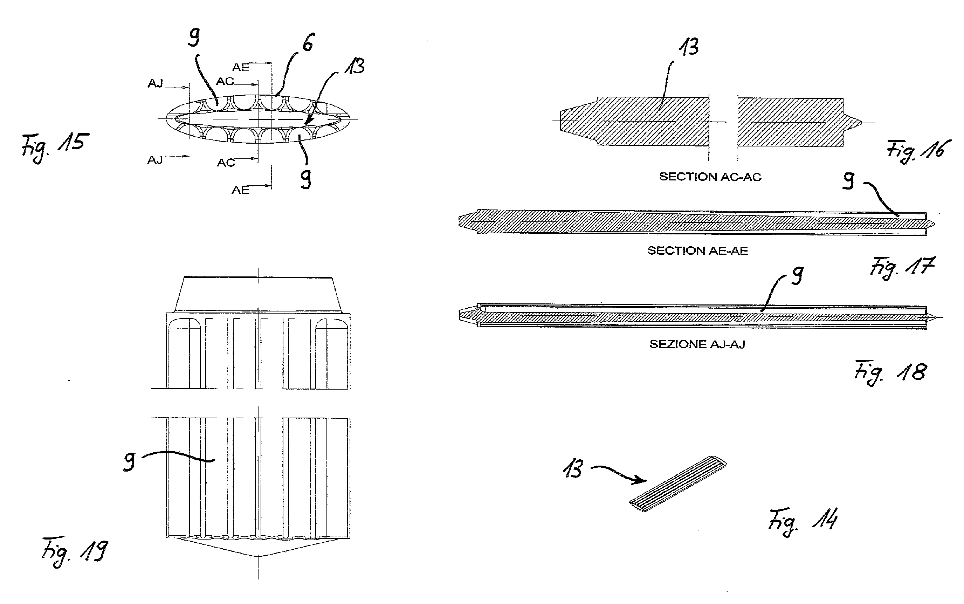

In einem abgewandelten Ausführungsbeispiel des Einsatzes, welche in den Figuren 14 - 19 dargestellt ist, sind die Strömungskanäle 9 so geformt, dass sich ihr Strömungsquerschnitt in Strömungsrichtung kontinuierlich verringert. Dies ist insbesondere in den Figuren 17 und 18 zu erkennen. Durch diese Ausformung der Strömungskanäle wird erreicht, dass die Stromdichte des aus der Filtereinheit 4 in die Kammer 7 strömenden Fluids über die gesamte Länge der Filterkerze 4a zumindest im Wesentlichen konstant ist.

In dem weiteren Ausführungsbeispiel eines Einsatzes 13, welches in den Figuren 14 - 19 dargestellt ist, sind die Strömungskanäle 9 dagegen mit gleichbleibendem Strömungsquerschnitt über die gesamte Länge des Einsatzes 13 geformt. Bei diesem Ausführungsbeispiel ist die Stromdichte das aus der Filtereinheit 4 strömenden Fluids im strömungsabwärtigen (hinteren) Bereich geringer als im strömungsaufwärtigen (vorderen) Bereich.

## Patentansprüche

1. Druckmesszelle zur Verwendung in einem Infusions- oder Injektionssystem zur Injektion eines Fluids, mit einem Gehäuse (1), in dem wenigstens eine Filtereinheit (4), durch welche das unter Druck stehende Fluid geleitet wird, sowie wenigstens ein Druckaufnehmer (3) zur Erfassung des Drucks des aus der Filtereinheit (4) strömenden Fluids angeordnet sind.

2. Druckmesszelle nach Anspruch 1, **dadurch gekennzeichnet, dass** die Filtereinheit (4) als längliche Filterkerze (4a) mit einem Kerzengehäuse (5) und einem Filtersieb (6) ausgebildet ist, wobei das Kerzengehäuse (5) eine Kerzenöffnung (8) aufweist, durch welche das unter Druck stehende Fluid in die Filterkerze (4a) einströmt.

3. Druckmesszelle nach Anspruch 2, **dadurch gekennzeichnet, dass** die Filterkerze (4a) in einer Gehäusebohrung (1c) angeordnet ist und dass zwischen der Innenfläche der Gehäusebohrung (1c) und der Außenseite der in das Gehäuse (1) eingesteckten Filterkerze (4a) eine mit der Auslassöffnung (2b) in Verbindung stehende Kammer (7) ausgebildet ist, in welche das Fluid aus der Filtereinheit (4) strömt.

4. Druckmesszelle nach Anspruch 2 oder 3, **dadurch gekennzeichnet, dass** das Kerzengehäuse (5) das Filtersieb (6) trägt und im Kerzengehäuse (5) vom Filtersieb (6) abgedeckte Ausnehmungen ausgebildet sind, durch welche das Fluid aus der Filtereinheit (4) ausströmt.

5. Druckmesszelle nach einem der Ansprüche 2 bis 4, **dadurch gekennzeichnet, dass** das Filtersieb (6) aus einem Filtergewebe mit einer Maschenweite von 10 - 15 µm gebildet ist, wobei das Filtergewebe hydrophil oder hydrophob ist.

6. Druckmesszelle nach einem der Ansprüche 2 bis 5, **dadurch gekennzeichnet, dass** die Stromdichte des aus der Filtereinheit (4) ausströmenden Fluids längs der Filterkerze (4a) im Wesentlichen konstant ist.

7. Druckmesszelle nach einem der Ansprüche 2 bis 5, **dadurch gekennzeichnet, dass** im Kerzengehäuse (5) in Längsrichtung der Filterkerze (4a) verlaufende Strömungskanäle (9) ausgebildet sind, in denen das Fluid geführt wird.

8. Druckmesszelle nach Anspruch 7, **dadurch gekennzeichnet, dass** die Strömungskanäle (9) einerseits von der Außenfläche des Kerzengehäuses (5) und andererseits vom Filtersieb (6) begrenzt sind.

9. Druckmesszelle nach Anspruch 7 oder 8, **dadurch gekennzeichnet, dass** sich der Strömungsquerschnitt der Strömungskanäle (9) in Strömungsrichtung (v) kontinuierlich verringert.

10. Druckmesszelle nach einem der voranstehenden Ansprüche, **dadurch gekennzeichnet, dass** der oder jeder Druckaufnehmer (3) durch eine Membran (10) gebildet ist, auf welche der Druck des Fluids einwirkt.

11. Druckmesszelle nach einem der voranstehenden Ansprüche, **dadurch gekennzeichnet, dass** der oder jeder Druckaufnehmer (3) mit einem Drucksensor, insbesondere einem piezoresistiven oder einem piezoelektrischen Drucksensor, koppelbar ist.

12. Druckmesszelle nach einem der voranstehenden Ansprüche, **dadurch gekennzeichnet, dass** die Filtereinheit (4) mittels einem in die Einlassöffnung (2a) einschiebbaren Stopfen (11) mit einem Anschlußstück (12) für eine Schlauchleitung des Infusions- oder Injektionssystem in dem Gehäuse (1) gesichert ist.

13. Druckmesszelle nach einem der voranstehenden Ansprüche, **dadurch gekennzeichnet, dass** die Filtereinheit (4) und die Innenfläche des Gehäuses (1) über miteinander korrespondierende Führungsmittel (22, 23), insbesondere über Führungsnuten (23) und Führungsrippen (22) verfügen.

14. Druckmesszelle nach einem der voranstehenden Ansprüche, **dadurch gekennzeichnet, dass** die Filtereinheit (4) austauschbar in einer Gehäusebohrung (1c) angeordnet ist.
